# EUROPEAN PATENT APPLICATION

(11) **EP 4 609 816 A1**
(43) Date of publication of application: **03.09.2025**
(21) Application number: 23893716.3
(22) Date of filing: 16.11.2023
(51) Int. Cl.: A61B 34/30

(54) **STERILE ADAPTER**

(30) Priority: 25.11.2022 CN 202211489899
(71) Applicant: Cornerstone Technology (Shenzhen) Limited, Shenzhen Guandong 518066 (CN)
(72) Inventor: YANG, Qiusheng, Shenzhen, Guangdong 518066 (CN); XU, Aoxiang, Shenzhen, Guangdong 518066 (CN); WANG, Zerui, Shenzhen, Guangdong 518066 (CN)
(74) Representative: Valet Patent Services Limited
(86) International application number: PCT/CN2023/132097
(87) International publication number: WO 2024/109626

(57) **Abstract**

Provided is a sterile adapter configured to connect a surgical instrument (14) to an instrument drive (13). The sterile adapter includes an input member (30), an output member (40) and an elastic member (50). The input member (30) is configured to be connected to the instrument drive (13) and receive a torque output from the instrument drive (13). The output member (40) is connected to the input member (30) and movable relative to the input member (30) in a first direction. The output member (40) is configured to be connected to the surgical instrument (14) and to transmit a torque between the input member (30) and the surgical instrument (14). An accommodating cavity (301) is formed between the input member (30) and the output member (40) and has a first end adjacent to the instrument drive (13) and a second end adjacent to the surgical instrument (14), and at least one of the first end and the second end is sealed. The elastic member (50) is accommodated in the accommodating cavity (301) and configured to provide, to the input member (30) and the output member (40), a restoration force moving the input member (30) and the output member (40) away from each other in the first direction. In the sterile adapter (20) in the present disclosure, the drive vacancy between the sterile adapter (20) and the instrument drive (13) and the surgical instrument (14) is reduced, improving the drive efficiency.

## Description

### CROSS REFERENCE TO RELATED APPLICATIONS

This application claims priority to Chinese Patent Application No. CN202211489899.9, entitled "STERILE ADAPTER," filed on November 25, 2022, which is incorporated by reference herein in its entirety.

### TECHNICAL FIELD

The present disclosure belongs to the technical field of medical instruments, and particularly relates to a sterile adapter.

### BACKGROUND

A surgical robot is a robot that can be manipulated remotely to complete surgical procedures and includes three components: a console, a robotic arm system, and an imaging system. The robotic arm system has an instrument drive mounted on an end link of the articulated arm of the robotic arm system, and a surgical instrument or an endoscope is operably mounted on the instrument drive. The surgical instrument includes three parts, i.e., a rear end mechanism, a main shaft extending from the rear end mechanism to the front end, and an end effector including a wrist mechanism at a front end of the main shaft. Generally, the rear end mechanism is driven by the instrument drive to move a plurality of cables in the main shaft, and then the wrist mechanism is driven with motions of the plurality of cables in the main shaft. During surgery, the wrist mechanism and a part of the main shaft of the surgical instrument pass through tissue such as the thoracic wall or abdominal wall to perform surgery instead of a human hand.

The surgical instruments mounted on the instrument drive on a surgery side is controlled by a surgeon on a console side. In order to meet different requirements for surgical instruments during surgery, the surgical instruments and the instrument drive are usually designed to be detachable for replacement of surgical instruments during surgery, and the surgical instruments are usually sterilizable independently of each other. An instrument drive end is usually designed to be non-sterilizable. In order to ensure the sterility of the surgical process, during surgery, it is necessary to add employ a sterile adapter between the instrument drive and an instrument, so as to isolate a non-sterilizable instrument drive from the sterilizable instrument during the surgery.

The existing sterile adapters have unstable connection with the instrument drive and the surgical instruments, have low drive efficiency, and are prone to the problem of drive vacancy or even motion jamming.

### SUMMARY

Embodiments of the present disclosure provide a sterile adapter, which is capable of improving the drive efficiency.

Embodiments of the present disclosure provide a sterile adapter configured to connect a surgical instrument to an instrument drive. The sterile adapter includes an input member, an output member, and an elastic member. The input member is configured to be connected to the instrument drive and to receive a torque output by the instrument drive. The output member is connected to the input member and movable relative to the input member in a first direction. The output member is configured to be connected to the surgical instrument and to transmit a torque between the input member and the surgical instrument. An accommodating cavity is formed between the input member and the output member and has a first end adjacent to the instrument drive and a second end adjacent to the surgical instrument, and at least one of the first end and the second end is sealed. The elastic member is accommodated in the accommodating cavity and configured to provide, to the input member and the output member, a restoration force moving the input member and the output member away from each other in the first direction.

According to implementations of a first aspect of the present disclosure, the input member includes a first bottom and a first sleeve protruding from the first bottom, and the output member includes a second bottom and a second sleeve protruding from the second bottom, the second sleeve and the first sleeve being fitted onto one another and defining the accommodating cavity.

According to implementations of the first aspect of the present disclosure, the drive assembly further includes an anti-disengagement assembly. The anti-disengagement assembly includes a limiting portion and a stopping portion. The limiting portion is disposed on one of the input member and the output member, and the stopping portion is disposed on the other one of the input member and the output member. Each of the limiting portion and the stopping portion are configured to prevent or stop the other one of them from moving in the first direction.

According to implementations of the first aspect of the present disclosure, the input member further includes a third sleeve protruding from the first bottom. The third sleeve is located within the first sleeve and defines a limiting space. The first bottom defines an opening communicated with the third sleeve, the third sleeve is provided with a limiting plate on a side of the third sleeve facing away from the first bottom, and the limiting plate defines an opening. The output member further includes a fourth sleeve protruding from the second bottom and extending into the limiting space through the opening of the limiting plate. The fourth sleeve is movable relative to the limiting plate in the first direction, and defines a catch space and a catch opening communicated with the catch space. The drive assembly further includes a catch member. The catch member includes a connecting portion and the stopping portion disposed at one end of the connecting portion in the first direction. The connecting portion is located in the catch space, and the stopping portion is stopped from moving by the limiting plate.

According to implementations of the first aspect of the present disclosure, the elastic member is sleeved over the fourth sleeve and the third sleeve.

According to implementations of the first aspect of the present disclosure, the catch member is threaded to the fourth sleeve.

According to implementations of the first aspect of the present disclosure, the fourth sleeve and the second sleeve protrude from the second bottom on a same side of the second bottom.

According to implementations of the first aspect of the present disclosure, the drive assembly further includes a limiting mechanism formed by a portion of an outer wall surface of the first sleeve and a portion of an inner wall surface of the second sleeve that are mated with each other. Neither the outer wall surface of the first sleeve nor the inner wall surface of the second sleeve is cylindrical. The portion of the limiting mechanism located on the outer wall surface of the first sleeve and mated/contacted with the inner wall surface of the second sleeve has a same cross-section at any position in the first direction, and the portion of the limiting mechanism located on the inner wall surface of the second sleeve and mated/contacted with the outer wall surface of the first sleeve has a same cross-section at any position in the first direction.

According to implementations of the first aspect of the present disclosure, the limiting mechanism includes a limiting groove and a limiting block. The limiting groove is disposed on one of the outer wall surface of the first sleeve and the inner wall surface of the second sleeve, and the limiting block is disposed on the other one of the outer wall surface of the first sleeve and the inner wall surface of the second sleeve. The limiting block is located in the limiting groove and movable relative to the limiting groove in the first direction.

According to implementations of the first aspect of the present disclosure, a plurality of limiting mechanisms are provided, and the plurality of the limiting mechanisms are arranged at intervals around an axis of the first sleeve.

According to implementations of the first aspect of the present disclosure, the drive assembly further includes an orientation mechanism formed by a portion of the outer wall surface of the first sleeve and a portion of the inner wall surface of the second sleeve that are mated with each other. The orientation mechanism has a predetermined angle or position with at least one of a drive input end or a drive output end of the drive assembly, and the portion of the orientation mechanism located on the outer wall surface of the first sleeve and mated/contacted with the inner wall surface of the second sleeve has a same cross-section at any position in the first direction, and the portion of the orientation mechanism located on the inner wall surface of the second sleeve and mated/contacted with the outer wall surface of the first sleeve has a same cross-section at any position in the first direction.

According to implementations of the first aspect of the present disclosure, the orientation mechanism includes an orientation groove and an orientation block. The orientation groove is disposed on one of the outer wall surface of the first sleeve and the inner wall surface of the second sleeve, and the orientation block is disposed on the other one of the outer wall surface of the first sleeve and the inner wall surface of the second sleeve. The orientation block is located in the orientation groove and movable relative to the orientation groove in the first direction.

According to implementations of the first aspect of the present disclosure, the input member further includes a drive disk. The drive disk is disposed on a surface of the first bottom facing away from the first sleeve, connected to an edge of the first bottom, protruding in a direction away from the first sleeve, and configured to connect the instrument drive and receive the torque output from the instrument drive.

According to implementations of the first aspect of the present disclosure, at least one of a surface of the second bottom facing the first bottom or a surface of the first bottom facing the second bottom is provided with a positioning groove, and the elastic member is limited within the positioning groove.

According to implementations of the first aspect of the present disclosure, the sterile adapter includes a first base body and a second base body. The first base body is fixed to the second base body in a stacked manner, a mounting hole is provided on the first base body and the second base body and penetrates the first base body and the second base body, the mounting hole has a first flange at the first base body and a second flange at the second base body, the drive assembly is mounted in the mounting hole, an outer periphery of the drive assembly is provided with a catch tab, the catch tab is located in a space formed by the first flange and the second flange, and a radius of a circumference at which the catch tab is located is greater than a radius of each of the first flange and the second flange.

According to implementations of the first aspect of the present disclosure, the outer periphery of the drive assembly is provided with at least two catch tabs, and a connecting line between the at least two catch tabs passes through a drive axis of the drive assembly.

According to implementations of the first aspect of the present disclosure, the outer periphery of the drive assembly is provided with two catch tabs, and the two catch tabs are different in size or arc length.

According to implementations of the first aspect of the present disclosure, a calibration block is disposed in the mounting hole between the first flange and the second flange, the calibration block is adjacent or proximate to the first flange, and a distance between the calibration block and the second flange is greater than a thickness of the catch tab.

The sterile adapter provided in the present disclosure is connected to the instrument drive through the input member and receives the torque output by the instrument drive, and is connected to the surgical instrument through the output member and transmits the torque to the surgical instrument. Isolation of the instrument drive from the surgical instrument is achieved with one end of the accommodating cavity sealed. The input member is connected to the output member and movable relative to the output member in the first direction to allow movement of the input member and the output member with respect to each other, which facilitates the connection of the input member with the instrument drive and the connection of the output member with the surgical instrument. Tight abutment of the input member against the instrument drive in the axial direction and tight abutment of the output member against the surgical instrument in the axial direction are provided by the elastic member in the accommodating cavity, thereby reducing the drive vacancy between the sterile adapter, the instrument drive and the surgical instrument, and improving the drive efficiency.

### BRIEF DESCRIPTION OF THE DRAWINGS

To describe the technical solutions in the embodiments of the present disclosure more clearly, the following briefly introduces the accompanying drawings required for describing the embodiments. Those of ordinary skill in the art may still derive other accompanying drawings from these accompanying drawings without creative efforts.
FIG. 1 is a schematic view illustrating the structure of an exemplary surgical robot according to embodiments of the present disclosure.
FIG. 2 is a schematic view illustrating the structure of an exemplary robotic arm system according to embodiments of the present disclosure.
FIG. 3 is a perspective view of an exemplary sterile adapter according to embodiments of the present disclosure.
FIG. 4 is a perspective view of an exemplary sterile adapter from another viewing angle according to embodiments of the present disclosure.
FIG. 5 a schematic view illustrating the exemplary structure of a surgical instrument, an instrument drive and a sterile adapter when assembled according to embodiments of the present disclosure.
FIG. 6 is a sectional view illustrating the exemplary structure of a surgical instrument, an instrument drive and a sterile adapter when assembled according to embodiments of the present disclosure.
FIG. 7 is a sectional view of a drive assembly, with an elastic member compressed, of an exemplary sterile adapter according to embodiments of the present disclosure.
FIG. 8 is a sectional view of a drive assembly, with an elastic member restored, of an exemplary sterile adapter according to embodiments of the present disclosure.
FIG. 9 is a sectional view illustrating the structure of a drive assembly of another exemplary sterile adapter according to embodiments of the present disclosure.
FIG. 10 is a schematic view illustrating the structure of an exemplary input member according to embodiments of the present disclosure.
FIG. 11 is a schematic view illustrating the structure of an exemplary output member according to embodiments of the present disclosure.

### DETAILED DESCRIPTION OF THE EMBODIMENTS

To make the objects, technical solutions and advantages of the present disclosure clearer, embodiments of the present disclosure are described in detail below in conjunction with the accompanying drawings. Those of ordinary skill in the art should appreciate that in embodiments of the present disclosure, numerous technical details have been presented to enable better understanding of the present disclosure. However, even without these technical details and various variations and modifications based on the following embodiments, the technical solutions claimed in the present disclosure can be realized.

In embodiments of the present disclosure, the terms "on," "below," "left," "right," "front," "back," "top," "bottom," "inside," "outside," "middle," "vertical," "horizontal," "transverse," "longitudinal," and the like indicate orientation or positional relationships based on the accompanying drawings. These terms are intended primarily to better describe the present disclosure and embodiments thereof and are not intended to limit that the indicated device element or component must have a particular orientation or be constructed and operated in a particular orientation.

Moreover, some of the above terms may be used to indicate other meanings in addition to the orientation or positional relationships. For example, the term "on" may also be used to indicate a certain attachment or connection relationship in some cases. For those of ordinary skill in the art, specific meanings of the terms in the present disclosure may be construed according to specific circumstances.

In addition, the terms "mounted," "disposed," "provided," "opened," "connected" and "connected to each other" should be understood in a broad sense. For example, the term "connected" may refer to "securely connected," "detachably connected" or "monolithically connected"; may refer to "mechanically connected" or "electrically connected"; and may refer to "connected directly," "connected indirectly through an intermediary" or "interconnected between two devices, elements or components". For those of ordinary skill in the art, specific meanings of the preceding terms in the present disclosure may be construed according to specific circumstances.

It is to be noted that as used herein, relationship terms such as "first" and "second" are used merely to distinguish one entity or operation from another, and does not necessarily require or imply any such actual relationship or order between these entities or operations. Furthermore, the terms "comprising," "including" or any other variant thereof are intended to encompass a non-exclusive inclusion so that a process, method, article or device that includes a series of elements not only includes the expressly listed elements but may further include other elements that are not expressly listed or are inherent to such process, method, article or device. In the absence of more restrictions, the elements defined by the statement "including ..." do not exclude the presence of additional identical elements in the process, method, article or device that includes the elements.

It is found that the existing sterile adapters have unstable connection with the instrument drive and the surgical instruments, have low drive efficiency, and are prone to the problem of drive vacancy or even motion jamming.

In view of the above problems, a sterile adapter is provided to connect a surgical instrument to an instrument drive. The sterile adapter includes a drive assembly. The drive assembly includes an input member, an output member and an elastic member. The input member is configured to be connected to the instrument drive and to receive a torque output from the instrument drive. The output member is connected to the input member and movable relative to the input member in a first direction, and the output member is configured to be connected to the surgical instrument and to transmit the torque between the input member to the surgical instrument, where an accommodating cavity is defined by the input member and the output member and has a first end adjacent to the instrument drive and a second end adjacent to the surgical instrument, and at least one of the first end and the second is sealed. The elastic member is accommodated in the accommodating cavity and configured to provide a restoration force to move the input member and the output member away from each other.

The sterile adapter provided in the present disclosure is connected to the instrument drive through the input member and receives the torque output by the instrument drive; and is connected to the surgical instrument through the output member and transmits the torque to the surgical instrument. Isolation of the instrument drive from the surgical instrument is achieved with one end of the accommodating cavity sealed. The input member is connected to the output member and movable relative to the output member in the first direction to allow movement of the input member and the output member with respect to each other, which facilitates the connection of the input member with the instrument drive and the connection of the output member with the surgical instrument. Tight abutment of the input member against the instrument drive in the axial direction and tight abutment of the output member against the surgical instrument in the axial direction are provided by the elastic member in the accommodating cavity, thereby reducing the drive vacancy between the sterile adapter, the instrument drive and the surgical instrument, and improving the drive efficiency.

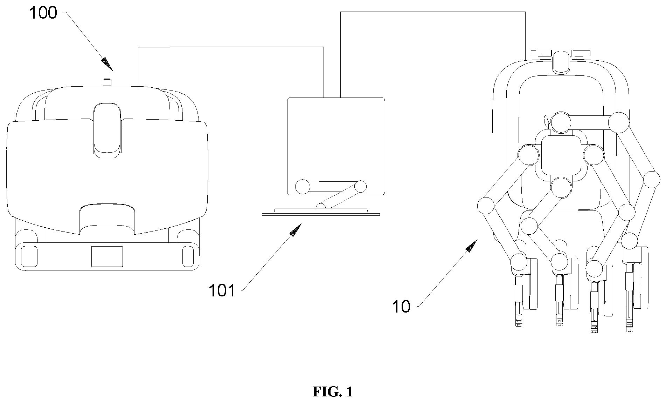

Referring to FIGS. 1 to 11, in order to solve the problems in the existing art, embodiments of the present disclosure provide a sterile adapter. The sterile adapter provided by the embodiments of the present disclosure is described below. Here, a direction annotated by X in the accompanying drawings indicates the first direction.

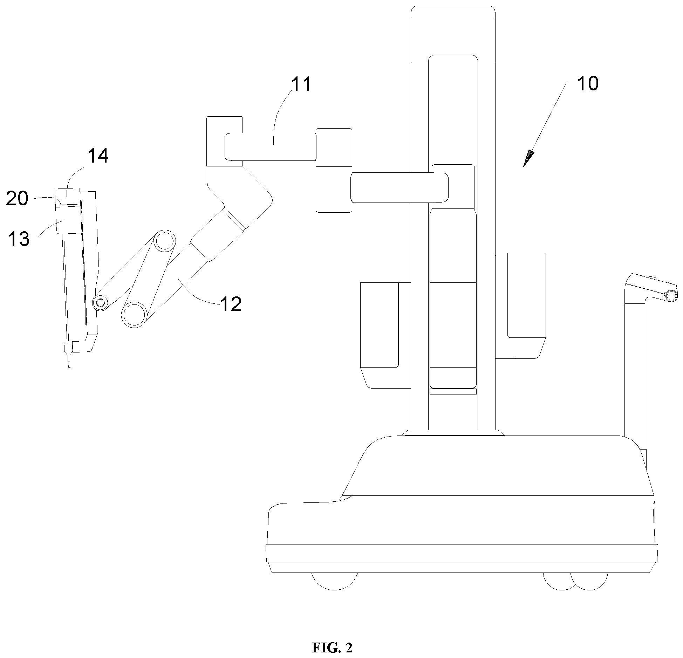

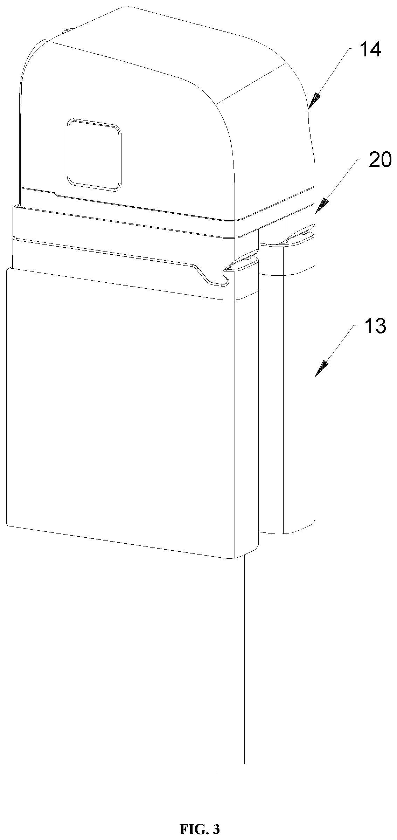

Referring to FIGS. 1 to 3, FIG. 1 is a schematic view illustrating the structure of an exemplary surgical robot according to embodiments of the present disclosure; FIG. 2 is a schematic view illustrating the structure of an exemplary robotic arm system according to embodiments of the present disclosure; and FIG. 3 is a perspective view of an exemplary sterile adapter according to embodiments of the present disclosure.

As shown in FIG. 1, the surgical robot includes a console 100, a robotic arm system 10 and an imaging system 101. The console 100 is provided with a display unit for displaying a working environment of the surgical instrument, an operation control mechanism and an armrest, where the display unit is provided with an observation window for observation by the surgeon, motions of the operation control mechanism effects motions of the surgical instrument, and the armrest is used for the surgeon's arms to rest on. In addition, there may be other control switches on the console 100 which are convenient for touching or pressing by a hand or foot to perform various functional operations so as to complete human-computer interaction. The imaging system 101 has a display screen, an endoscope controller, a system electronic equipment, an image processor, and the like.

As shown in FIG. 2, the robotic arm system 10 includes a set-up device 11, an operating device 12, an instrument drive 13, a surgical instrument 14, and a sterile adapter 20, where the set-up device 11 is connected to the operating device 12, and the instrument drive 13, the surgical instrument 14 and the sterile adapter 20 are connected to one end of the operating device 12.

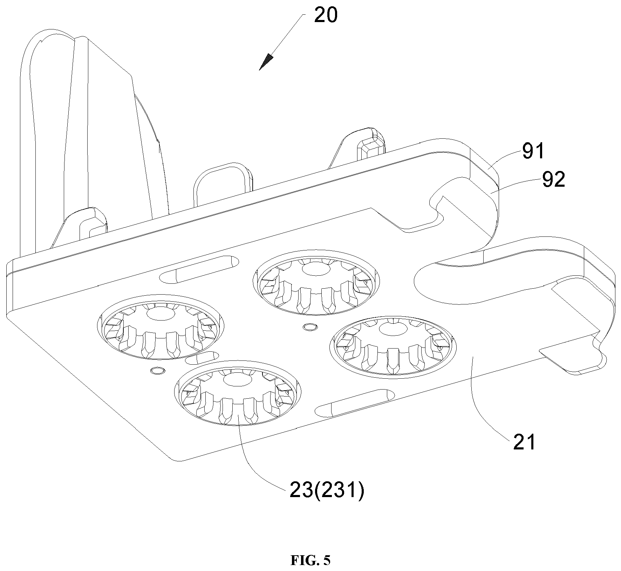

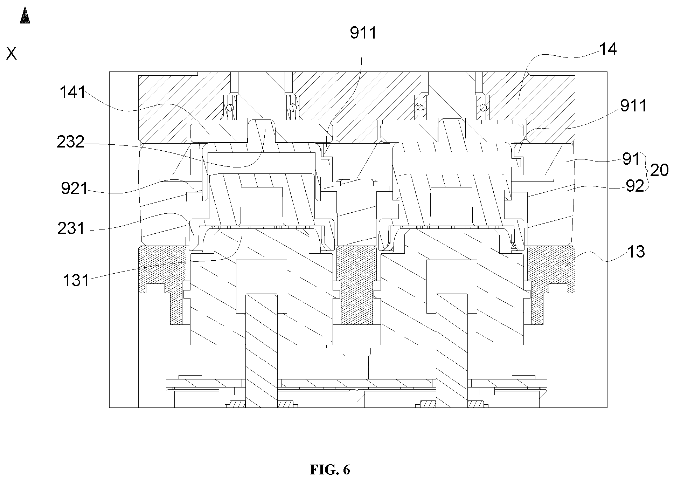

Referring to FIGS. 3 to 5, FIG. 4 is a perspective view of an exemplary sterile adapter from another viewing angle according to embodiments of the present disclosure; and FIG. 5 a schematic view illustrating the exemplary structure of a surgical instrument, an instrument drive and a sterile adapter according to embodiments of the present disclosure. The sterile adapter 20 has a first surface 21 engaging the instrument drive, and a second surface 22 engaging the surgical instrument. The first surface 21 and the second surface 22 are facing away from each other. The sterile adapter 20 further has a drive assembly 23 configured to drive the surgical instrument 14 to perform operations. In combination with FIG. 6 which is a sectional view illustrating the exemplary structure of a surgical instrument, an instrument drive and a sterile adapter when assembled according to embodiments of the present disclosure, an input end 231 of the drive assembly 23 of the sterile adapter 20 is coupled to a drive output end 131 of the instrument drive 13, and an output end 232 of the drive assembly 23 of the sterile adapter 20 is coupled to a drive input end 141 of the surgical instrument 14, so that the surgical instrument can be driven by the instrument drive to perform operations after assembly of the surgical instrument, the sterile adapter and the instrument drive.

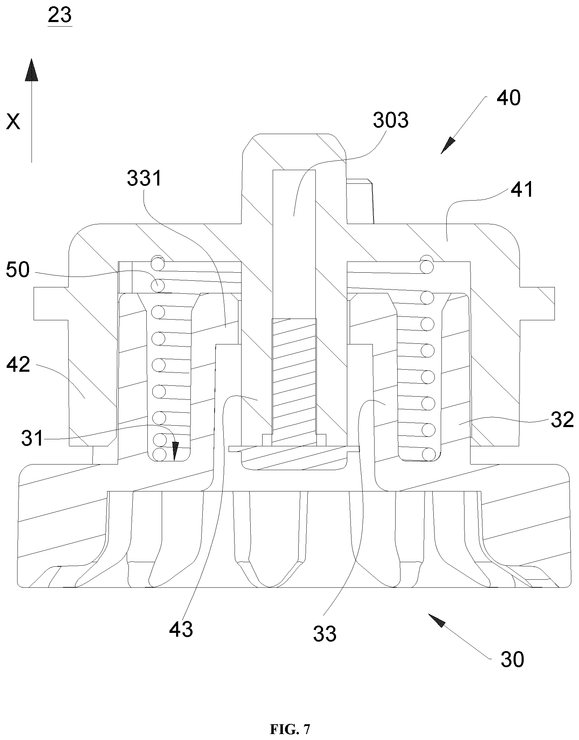

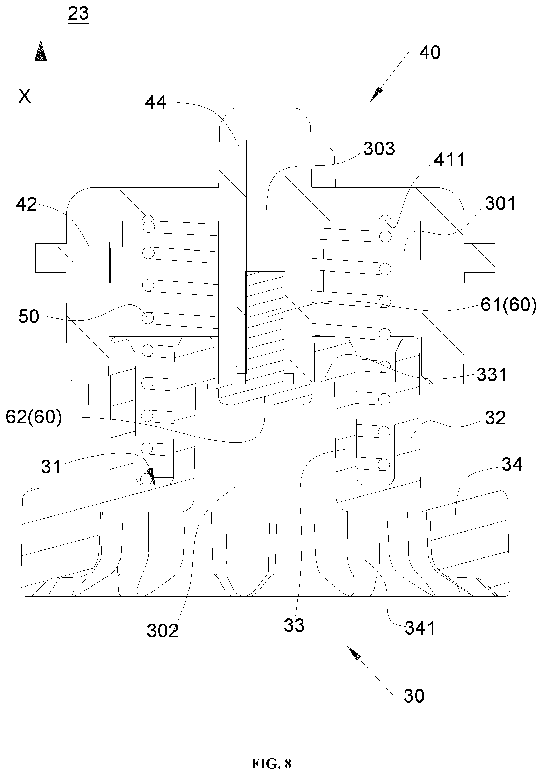

As shown in FIGS. 7 and 8, FIG. 7 is a sectional view of a drive assembly, with an elastic member compressed, of an exemplary sterile adapter according to embodiments of the present disclosure; and FIG. 8 is a sectional view of a drive assembly, with an elastic member restored, of an exemplary sterile adapter according to embodiments of the present disclosure. The drive assembly 23 includes an input member 30, an output member 40, and an elastic member 50. The input member 30 has a drive input end configured to be connected to the instrument drive 13 and to receive a torque output from the drive output end of the instrument drive 13. The output member 40 is connected to the input member 30 and movable relative to the input member 30 in a first direction (i.e., a direction annotated by "X" in the drawings, hereafter "X direction"), and has a drive output end configured to be connected to a drive input end of the surgical instrument 14 and to transmit the torque to the drive input end of the surgical instrument 14. An accommodating cavity 301 is defined by the input member 30 and the output member 40. The accommodating cavity 301 includes a first end (not designated) adjacent to the instrument drive 13 and a second end (not designated) adjacent to the surgical instrument 14. At least one of the first end and the second end is sealed. The elastic member 50 is located in the accommodating cavity 301, and is disposed between and abuts the input member 30 and the output member 40. The elastic member 50 is configured to provide a restoration force to move the input member 30 and the output member 40 away from each other.

The elastic member 50 may be a spring, or other elastic parts that can provide a restoration force, such as a tower spring, a gas spring, a spring sheet, or a block made from material with elasticity, such as rubber. The elastic member 50 may have a compressed state (as shown in FIG. 7) and a restored state (as shown in FIG. 8). As shown in FIG. 8, when the sterile adapter is not assembled with the surgical instrument 14 and the instrument drive 13, the elastic member is in a restored state. As shown in FIG. 7, when the sterile adapter is assembled with the surgical instrument 14 and the instrument drive 13, the drive member of the surgical instrument and the drive member of the instrument drive press the output member and the input member of the sterile adapter respectively, and the elastic member 50 is in a compressed state.

The drive assembly of the sterile adapter provided in the embodiments is connected to the instrument drive 13 through the input member 30 and receives the torque output by the instrument drive 13; and is connected to the surgical instrument 14 through the output member 40 and transmits the torque to the surgical instrument 14. Isolation of the instrument drive 13 from the surgical instrument 14 is achieved with at least one end of the accommodating cavity 301 sealed. The input member 30 is connected to the output member 40 and movable relative to the output member 40 in the first direction (i.e., the X direction in the drawings) to allow movement of the input member 30 and the output member 40 with respect to each other, which facilitates the connection of the input member 30 with the instrument drive 13 and the connection of the output the connection of 40 with the surgical instrument 14. Tight abutment of the input member 30 against the instrument drive 13 in the axial direction and tight abutment of the output member 40 against the surgical instrument 14 in the axial direction are provided by the elastic member 301 in the accommodating cavity 301, thereby reducing the drive vacancy between the sterile adapter 20, the instrument drive 13 and the surgical instrument 14, and improving the drive efficiency.

In some optional embodiments, the input member 30 includes a first bottom 31 and a first sleeve 32. The first sleeve 32 protrudes from the first bottom 31. The output member 40 includes a second bottom 41 and a second sleeve 42. The second sleeve 42 protrudes from the second bottom 41, and the second sleeve 42 and the first sleeve 32 are fitted onto one another and defines the accommodating cavity 301. The elastic member 50 is located in the accommodating cavity 301, and is disposed between and abuts the first bottom 31 and the first bottom 31. The elastic member 50 is configured to provide a restoration force to move the input member 30 and the output member 40 away from each other.

The first direction (i.e., the X direction in the drawings) is a direction of a connecting line between the first bottom 31 and the second bottom 41. The first sleeve 32 and the second sleeve 42 are located between the first bottom 31 and the second bottom 41. Axes of the first sleeve 32 and the second sleeve 42 are parallel to the first direction (i.e., the X direction in the drawings).

In the drive assembly of the sterile adapter provided in the embodiments, the first sleeve 32 and the second sleeve 42 respectively protrude from the first bottom 31 and the second bottom 41. The first sleeve 32 and the second sleeve 42are fitted onto one another, which prevents relative movement between the input member 30 and the output member 40 in the radial direction, and achieves sealing of both ends of the accommodating cavity 301, to isolate the instrument drive 13 from the surgical instrument 14, and to prevent foreign matter from entering the sterile adapter 20 to affect the movement of the sterile adapter 20. The first sleeve 32 and the second sleeve 42 define the accommodating cavity 301, the elastic member 50 is located in the accommodating cavity 301, and is disposed between and abuts the first bottom 31 and the second bottom 41. Tight abutment of the first bottom 31 against the instrument drive 13 in the axial direction and tight abutment of the second bottom 41 against the surgical instrument 14 in the axial direction are provided by the elastic member 301, thereby reducing the drive vacancy between the sterile adapter 20, the instrument drive 13 and the surgical instrument 14, and improving the drive efficiency.

Reference is made to FIG. 8 which is a sectional view of a drive assembly, with an elastic member in a restored state, of an exemplary sterile adapter according to embodiments of the present disclosure.

In some optional embodiments, the drive assembly 23 of the sterile adapter 20 may further include an anti-disengagement assembly. The anti-disengagement assembly includes a limiting portion and a stopping portion 62. The limiting portion may be provided on one of the input member 30 and the output member 40, and the stopping portion 62 may be provided on the other one of the input member 30 and the output member 40. Each of the limiting portion and the stopping portion 62 prevents or stops the other one of them from moving in the X direction to prevent disengagement of the input member from the output member in the X direction.

Specifically, in one embodiment, as shown in FIGS. 7 and 8, the input member 30 includes a third sleeve 33 which protrudes from the first bottom 31 and is integrally formed with the first bottom 31. The third sleeve 33 is located within the first sleeve 32 and defines a limiting space 302. The first bottom 31 is provided with an opening (in which a member is mounted) communicating with the third sleeve 33, and a limiting plate 331 is provided on a side of the third sleeve 33 facing away from the first bottom portion 31, and is provided with an opening. The output member 40 may further include a fourth sleeve 43 protruding from the second bottom 41. The fourth sleeve 43 extends into the limiting space 302 through the opening of the limiting plate 331 and is movable in a first direction (i.e., the X direction in the drawings) with respect to the limiting plate 331. The fourth sleeve 43 defines a catch space 303 and a catch opening communicating with the catch space 303. The sterile adapter 20 may further include a catch member 60. The catch member 60 includes a connecting portion 61 and a stopping portion 62. The connecting portion 61 extends into the catch space 303 from the catch opening. The stopping portion 62 is disposed at one end of the connecting portion 61 in the first direction (i.e., the X direction in the drawings) and is stopped from moving by the limiting plate 331.

The third sleeve 33 and the fourth sleeve 43 are located between the first bottom 31 and the second bottom 41. Axes of the third sleeve 33 and the fourth sleeve 43 are parallel to the first direction (i.e., the X direction in the drawings). The stopping portion 62 is located in the limiting space 302. In the sterile adapter 20 provided in this embodiment, the third sleeve 33 and the fourth sleeve 43 are fitted onto one another and movable in the first direction (i.e., the X direction in the drawings) relative to each other so that relative movement between the input member 30 and the output member 40 in the radial direction is further prevented. Disengagement between the third sleeve 33 and the fourth sleeve 43 is prevented with the limiting plate 331 provided on the side of the third sleeve 33 facing away from the first bottom 31 and the stopping portion 62 stopped by the limiting plate 331, so that the input member 30 and the output member 40 are movable in the first direction (i.e., the X direction in the figure) relative to each other without a risk of disengagement therebetween. In some optional embodiments, the elastic member 50 is sleeved over the fourth sleeve 43 and the third sleeve 33, that is, the elastic member 50, the fourth sleeve 43 and the third sleeve 33 are all located in the accommodating cavity 301, and the elastic member 50 is located between one of the first and second sleeves 32, 42 and one of the third and fourth sleeves 33, 43.

In some optional embodiments, the catch member 60 is threaded to the fourth sleeve 43. The connecting portion 61 is provided with an external thread, and the inner wall surface of the fourth sleeve 43 is provided with an internal thread mated with the external thread on the connecting portion 61.

According to the sterile adapter 20 provided in this embodiment, the catch member 60 and the fourth sleeve 43 are synchronously moved through the threaded connection of the catch member 60 to the fourth sleeve 43, and the stopping portion 62 on the catch member 60 is caught and stopped by the limiting plate 331, so that the input member 30 and the output member 40 are movable in the first direction (i.e., the X direction in the drawings) relative to each other without a risk of disengagement therebetween.

In some optional embodiments, the fourth sleeve 43 and the second sleeve 42 protrude from the same side of the second bottom 41.

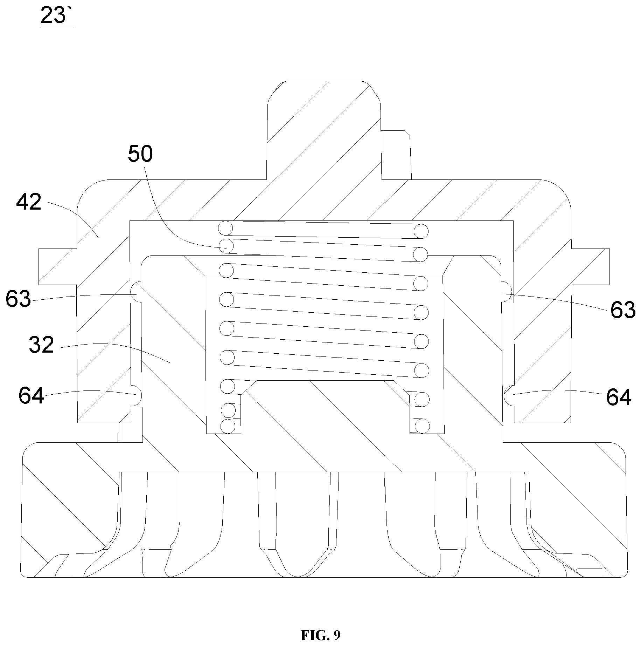

Reference is made to FIG. 9 which is a sectional view illustrating the structure of a drive assembly of another exemplary sterile adapter according to embodiments of the present disclosure.

FIG. 9 shows a drive assembly 23' in another embodiment. In some embodiments, the anti-disengagement assembly may include a rib structure and a bump structure with one of them provided on the first sleeve 32 and the other of them provided on the second sleeve 42. In one embodiment, a rib 63 is disposed on the outer surface of the first sleeve 32, and several bumps 64 are disposed on the inner surface of the second sleeve 42. An inner diameter of the rib 63 is slightly smaller than an outer diameter of a virtual ring formed by the bumps 64. In the assembly of the input member 30 and the output member 40, the input member 30 and the output member 40 are strongly pressed by an external force, and the rib 63 and the bumps 64 are slightly deformed as a result of the characteristics of the materials of the input member and the output member, so that the first sleeve 32 and the second sleeve 42 break through the restriction of the rib 63 and the bumps 64 to complete the assembly. After the first sleeve 32 and the second sleeve 42 are assembled, the elastic force of the elastic member is insufficient to cause deformation of the rib and the bumps, so that the rib and the bumps can prevent movement relative to each other. In this way, the first sleeve 32 and the second sleeve 42 are movable in the first direction (i.e., the X direction in the drawings) relative to each other without a risk of disengagement therebetween, so as the input member 30 and the output member 40. In some embodiments, the rib structure may be disposed on the inner surface of the second sleeve, and the bump structure may be disposed on the outer surface of the first sleeve. In some embodiments, the anti-disengagement assembly may include two rib structures, that is, each of the first sleeve and the second sleeve is provided with a rib structure. In some embodiments, the anti-disengagement assembly may include two bump structures, that is, each of the first sleeve and the second sleeve is provided with a bump structure, which can also prevent disengagement.

In some embodiments, two ends of the elastic member 50 are fixedly connected to the first bottom 31 and the second bottom 41 respectively, for example, by interference fit, welding or bonding to prevent the input member 30 and the output member 40 from being disengaged from each other, and the elastic member 50 provides a restoration force to the input member 30 and the output member 40 to keep them away from each other in the first direction (i.e., the X direction in the drawings) but without a risk of disengagement therebetween.

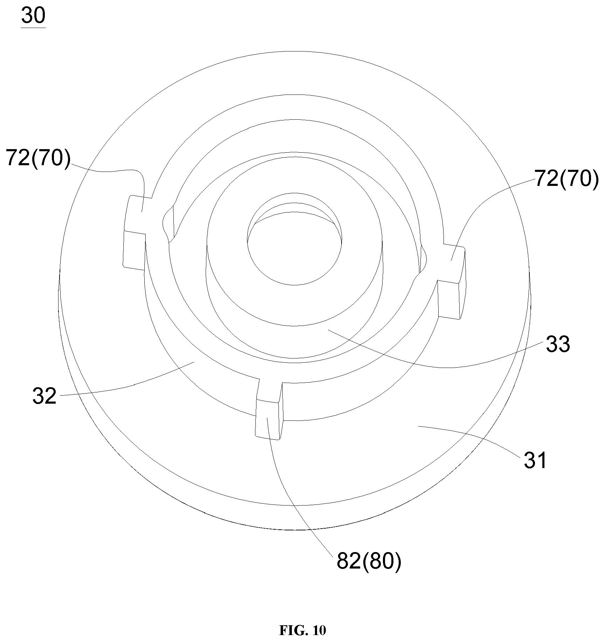

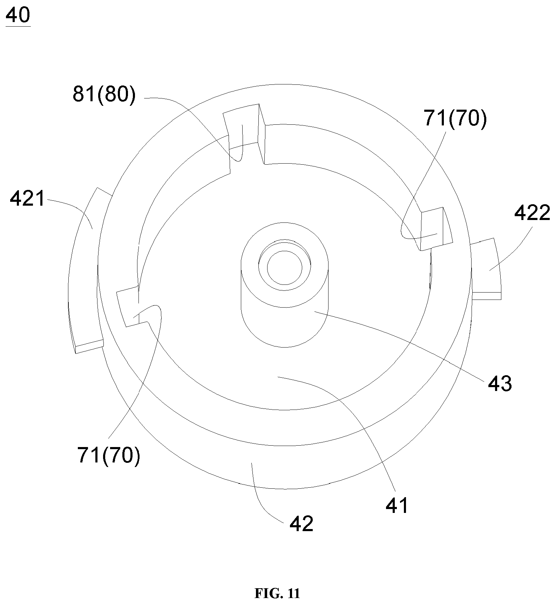

Referring to FIGS. 10 and 11, FIG. 10 is a schematic view illustrating the structure of an exemplary input member, and FIG. 11 is a schematic view illustrating the structure of an exemplary output member.

As shown in FIGS. 10 and 11, in some optional embodiments, the sterile adapter 20 may further include a limiting mechanism 70. The limiting mechanism 70 includes a limiting groove 71 and a limiting block 72, where the limiting groove 71 is disposed on one of the outer wall surface of the first sleeve 32 and the inner wall surface of the second sleeve 42, and the limiting block 72 is disposed on the other one of the outer wall surface of the first sleeve 32 and the inner wall surface of the second sleeve 42. The limiting block 72 is located in the limiting groove 71 and movable relative to the limiting groove 71 in the first direction.

The above arrangement applies to a case of the first sleeve 32 being located inside the second sleeve 42. In a further case of the first sleeve 32 being located outside the second sleeve 42, the limiting groove 71 is provided on one of an inner wall surface of the first sleeve 32 and an outer wall surface of the second sleeve 42, and the limiting block 72 is provided on the other one of the inner wall surface of the first sleeve 32 and the outer wall surface of the second sleeve 42.

In the embodiments, the limiting groove 71 makes the outer wall surface of the first sleeve 32 not a complete cylindrical surface, and the limiting block 72 makes the inner wall surface of the second sleeve 42 not a complete cylindrical surface. In some embodiments, the inner shape of the first sleeve 32 may be polygonal, irregularly shaped, or the like, and the outer shape of the second sleeve 42 may also be polygonal, irregularly shaped, or the like. The inner wall surface of the first sleeve may be in full fit/contact with the outer wall surface of the second sleeve. Alternatively, the inner wall surface of the first sleeve may be in partial fit/contact with the outer wall surface of the second sleeve, which results in a gap/void between the inner wall surface of the first sleeve and the outer wall surface of the second sleeve. For any of the foregoing cases, the portion where the inner wall surface of the first sleeve contacts the outer wall surface of the second sleeve has the same cross section at any position in the X direction. Therefore, the limiting mechanism is formed by a portion of the outer wall surface of the first sleeve and a portion of the inner wall surface of the second sleeve that are mated with each other in a case where neither the outer wall surface of the first sleeve nor the inner wall surface of the second sleeve is cylindrical. The portion of the limiting mechanism located on the outer wall surface of the first sleeve and mated/contacted with the inner wall surface of the second sleeve has the same cross-section at any position in the first direction, and the portion of the limiting mechanism located on the inner wall surface of the second sleeve and mated/contacted with the outer wall surface of the first sleeve has the same cross-section at any position in the first direction.

In the drive assembly 23 of the sterile adapter provided in this embodiment, the limiting groove 71 and the limiting block 72 are respectively disposed on the first sleeve 32 and the second sleeve 42, and are not rotatable relative to each other, but movable relative to each other in the first direction (i.e., the X direction in the drawings), so that a torque can be transmitted between the first sleeve 32 and the second sleeve 42, which enables torque transmission between the input member 30 and the output member 40.

In some optional embodiments, multiple limiting mechanisms 70 are provided and arranged at intervals around an axis of the first sleeve 32.

When two the limiting mechanisms 70 are provided, a straight line connecting center points of the two limiting mechanisms 70 intersects the axis of the first sleeve 32. When three limiting mechanisms 70 are provided, a first straight line and a second straight line intersect at an angle of 120°, where the first straight line connects a center point of one of any two of the three limiting mechanisms 70 to the axis of the first sleeve 32, and the second straight line connects a center point of the other one of the any two of the three limiting mechanisms 70 to the axis of the first sleeve 32. And so on for more limiting mechanisms 70. In the sterile adapter 20 provided in this embodiment, the multiple limiting mechanisms 70 are provided at intervals so that the drive vacancy between the sterile adapter 20 and the instrument drive 13 and the surgical instrument 14 is reduced, thereby improving the drive efficiency.

In some optional embodiments, the drive assembly 23 of the sterile adapter further includes an orientation mechanism 80 which includes an orientation groove 81 and an orientation block 82. The orientation groove 81 is provided on one of the outer wall surface of the first sleeve 32 and the inner wall surface of the second sleeve 42, and the orientation block 82 is provided on the other one of the outer wall surface of the first sleeve 32 and the inner wall surface of the second sleeve 42. The orientation block 82 is located within the orientation groove 81 and is movable relative to the orientation groove 81 in the first direction (i.e., the X direction in the drawings). A straight line connecting a center point of the orientation mechanism 80 to the axis of the first sleeve 32 and a straight line connecting a center point of the limiting mechanism 70 to the axis of the first sleeve 32 intersect at an acute or right angle.

The above arrangement applies to a case of the first sleeve 32 being located inside the second sleeve 42. In a further case of the first sleeve 32 being located outside the second sleeve 42, the orientation groove 81 is provided on one of the inner wall surface of the first sleeve 32 and the outer wall surface of the second sleeve 42, and the orientation block 82 is provided on the other one of the inner wall surface of the first sleeve 32 and the outer wall surface of the second sleeve 42.

In the embodiments, the orientation mechanism is formed by a portion of the outer wall surface of the first sleeve and a portion of the inner wall surface of the second sleeve that are mated with each other, and the orientation mechanism has a predetermined angle or position with at least one of the drive input end or the drive output end of the drive assembly. In addition, the portion of the orientation mechanism located on the outer wall surface of the first sleeve and mated/contacted with the inner wall surface of the second sleeve has the same cross-section at any position in the first direction, and the portion of the orientation mechanism located on the inner wall surface of the second sleeve and mated/contacted with the outer wall surface of the first sleeve has the same cross-section at any position in the first direction.

In the drive assembly 23 of the sterile adapter provided in this embodiment, the orientation mechanism 80 has a predetermined orientation or position relative to at least one of a connection key or a keyway of the drive output end or the drive input end, which ensures the consistency of the drive assembly 23 in batch processing. In addition, the orientation mechanism 80 can serve as a fool-proof structure, so that the first sleeve 32 and the second sleeve 42 have a unique assembly relationship in the circumferential direction, thereby improving the assembly efficiency.

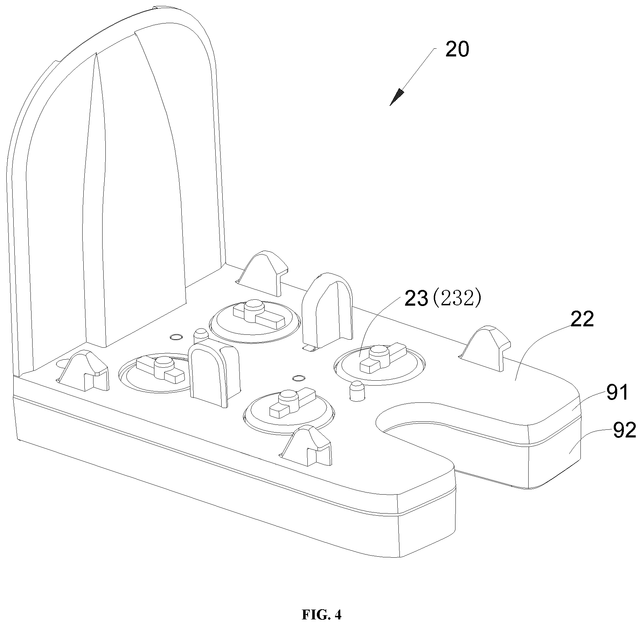

In conjunction with FIGS. 4, 5, 6 and 11, the sterile adapter 20 includes a first base body 91 and a second base body 92 that are stacked and fixedly connected to each other, with the first surface 21 at the first base body 91 and the second surface 22 at the second base body 92. A mounting hole (in which a member is mounted) is provided on the first base body 91 and the second base body 92 and penetrates the first base body 91 and the second base body 92. The drive assembly 23 is mounted in the mounting hole. In the mounting hole, a first flange 911 at an end of the first base body 91 adjacent to the first surface and a second flange 921 at an end of the second base body 92 connected to the first base body 91 are provided. Each of an inner diameter of the first flange 911 and an inner diameter of the second flange 921 is slightly larger than an outer diameter of the second sleeve 42. The second sleeve 42 is provided with a first catch tab 421 and a second catch tab 422 on the outer wall surface of the second sleeve 42. The first catch tab 421 and the second catch tab 422 are located in a same plane that is perpendicular to the X direction. Each of an edge of the first catch tab 421 and an edge of the second catch tab 422 is a part of a circle that is coaxial with the second sleeve 42, and a radius of the circle in which the edges of the first catch tab 421 and the second catch tab 422 are located is slightly greater than a radius of each of the first flange 911 and the second flange 921. When the drive assembly 23 is assembled in the mounting hole, the first flange 911 and the second flange 921 allows the first and second catch tabs 421 and 422 to be movable only in a space between the first flange 911 and the second flange 921, preventing the drive assembly 23 from going out of the mounting hole in a direction toward the first surface. A straight line connecting an edge of the first catch tab 421 to an edge of the second catch tab 422 intersects the axis of the second sleeve 42. The first catch tab 421 and the second catch tab 422 have different sizes and can be mated with a calibration block (which is mounted with a member) arranged in the mounting hole. The calibration block may be provided on the first base body, and after the sterile adapter is mounted on the instrument drive, the calibration block in a rotation plane of the catch tabs can limit the rotation of the first catch tab 421 and the second catch tab 422, so as to get a zero position calibrated for the instrument drive after the catch tab is rotated by a certain angle and then stopped by the calibration block. The first catch tab 421 and the second catch tab 422 have different sizes so that two circular arc segments between the two catch tabs have different lengths, thereby helping to find the zero position. In some embodiments, the calibration block is disposed adjacent or proximate to the first flange, with sufficient space between the calibration block and the second flange to accommodate the first catch tab 421 and the second catch tab 422. A distance between the calibration block and the second flange is greater than a thickness of each of the first catch tab 421 and the second catch tab 422, so that when an instrument is mounted on the sterile adapter, the drive assembly 23 is engaged with and pressed down by an input end of the surgical instrument, so that the drive assembly is released from stopping of the calibration block and rotates freely, thereby transmitting a force or a torque from an output shaft of a motor to an instrument end.

As shown in FIG. 8, in some optional embodiments, the second bottom 41 is provided with a connection key 44 on a surface of the second bottom 41 away from the first bottom 31, and the connection key 44 is configured to connect to the surgical instrument 14.

The sterile adapter 20 provided in this embodiment is connected to the surgical instrument 14 through the connection key 44 and transmits a torque to the surgical instrument 14, to improve the tightness of the connection between the output member 40 and the surgical instrument 14, which improves the drive efficiency between the sterile adapter 20 and the instrument drive 13.

In some optional embodiments, the input member 30 further includes a drive disc 34 disposed on a surface of the first bottom 31 facing away from the first sleeve 32. The drive disc 34 is connected to an edge of the first bottom 31 and protrudes in a direction away from the first sleeve 32. The drive disc 34 is provided with at least one keyway 341 for connecting with the instrument drive 13 and receiving a torque output from the instrument drive 13.

The drive assembly 23 of the sterile adapter provided in this embodiment is connected to the instrument drive 13 through the at least one keyway 341 on the drive disk 34 and receives a torque transmitted from the instrument drive 13, to improve the tightness of the connection between the input member 30 and the instrument drive 13, which improves the drive efficiency between the sterile adapter 20 and the instrument drive 13.

In some optional embodiments, as shown in FIG. 8, a surface of the second bottom 41 facing the first bottom 31 is provided with a positioning groove 411, and an end of the elastic member 50 is at least partially limited within the positioning groove 411 as shown in FIG. 8. For example, the elastic member 50 in the embodiment includes a spring, the shape of the positioning groove 411 formed along the surface of the second bottom may be annular, and a radius of the positioning groove 411 is the same as or slightly larger than a radius of a cross section of the spring. A width of a cross-section of the positioning groove 411 matches a width of the elastic member 50 so that the elastic member 50 can be limited within the positioning groove. In some embodiments, the positioning groove may be disposed on a surface of the first bottom 31 facing the second bottom 41. Alternatively, each of the second bottom 41 and the first bottom 31 may be provided with a positioning groove.

In the sterile adapter 20, the radial movement of the elastic member 50 in the accommodating cavity 301 is restricted by the positioning groove, which facilitates tight abutment of the first bottom 31 against the instrument drive 13 in the axial direction and tight abutment of the second bottom 41 against the surgical instrument 14, thereby improving the drive efficiency between the sterile adapter 20 and the instrument drive 13 and the surgical instrument 14.

The drive assembly 23 of the sterile adapter provided in this embodiment is connected to the instrument drive 13 through the input member 30 and receives a torque output from the instrument drive 13; and is connected to the surgical instrument 14 through the output member 40 and transmits the torque to the surgical instrument 14. Isolation of the instrument drive 13 from the surgical instrument 14 is achieved with at least one end of the accommodating cavity 301 sealed. The input member 30 is connected to the output member 40 and movable relative to the output member 40 in the first direction (i.e., the X direction in the drawings) to allow movement of the input member 30 and the output member 40 with respect to each other, which facilitates the connection of the input member 30 with the instrument drive 13 and the connection of the output the connection of 40 with the surgical instrument 14. Tight abutment of the input member 30 against the instrument drive 13 in the axial direction and tight abutment of the output member 40 against the surgical instrument 14 in the axial direction are provided by the elastic member 301 in the accommodating cavity 301, thereby reducing the drive vacancy between the sterile adapter 20, the instrument drive 13 and the surgical instrument 14, and improving the drive efficiency.

The above describes the prosthesis system provided by the embodiments of the present disclosure in detail, and specific examples are applied herein to illustrate the principles and embodiments of the present disclosure. The description of the above embodiments is only used to assist in the understanding of the ideas of the present disclosure, and there may be changes in the specific embodiments and the scope of the present disclosure. In conclusion, the contents of the specification should not be construed as limiting the present disclosure.

## Claims

1. A sterile adapter (20) configured to connect a surgical instrument (14) to an instrument drive (13), comprising:
a drive assembly (23), the drive assembly (23) including:
an input member (30) configured to be connected to the instrument drive (13) and to receive a torque output from the instrument drive (13);
an output member (40) connected to the input member (30) and movable relative to the input member (30) in a first direction, wherein the output member (40) is configured to be connected to the surgical instrument (14) and to transmit a torque between the input member (30) and the surgical instrument (14), an accommodating cavity (301) is formed between the input member (30) and the output member (40) and has a first end adjacent to the instrument drive (13) and a second end adjacent to the surgical instrument (14), and at least one of the first end and the second end is sealed; and
an elastic member (50) accommodated in the accommodating cavity (301) and configured to provide, to the input member (30) and the output member (40), a restoration force moving the input member (30) and the output member (40) away from each other in the first direction.

2. The sterile adapter (20) according to claim 1, wherein the input member (30) includes a first bottom (31) and a first sleeve (32) protruding from the first bottom (31);
wherein the output member (40) includes a second bottom (41) and a second sleeve (42) protruding from the second bottom (41), the second sleeve (42) and the first sleeve (32) being fitted onto one another and define the accommodating cavity (301); and
wherein the elastic member (50) is disposed between and abuts the first bottom (31) and the second bottom (41).

3. The sterile adapter (20) according to claim 2, wherein the drive assembly (23) further includes an anti-disengagement assembly, the anti-disengagement assembly includes a limiting portion and a stopping portion (62), the limiting portion is disposed on one of the input member (30) and the output member (40), the stopping portion (62) is disposed on the other one of the input member (30) and the output member (40), and each of the limiting portion and the stopping portion (62) are configured to prevent or stop the other one of them from moving in the first direction.

4. The sterile adapter (20) according to claim 3, wherein the input member (30) further includes a third sleeve (33) protruding from the first bottom (31), the third sleeve (33) is located within the first sleeve (32) and defines a limiting space (302), the first bottom (31) defines an opening communicated with the third sleeve (33), the third sleeve (33) is provided with a limiting plate (331) on a side of the third sleeve (33) facing away from the first bottom (31), and the limiting plate (331) defines an opening;
wherein the output member (40) further includes a fourth sleeve (43) protruding from the second bottom (41) and extending into the limiting space (302) through the opening of the limiting plate (331), the fourth sleeve (43) is movable relative to the limiting plate (331) in the first direction, and the fourth sleeve (43) defines a catch space (303) and a catch opening communicated with the catch space (303); and
wherein the drive assembly (23) further includes a catch member (60), the catch member (60) includes a connecting portion (61) and the stopping portion (62) disposed at one end of the connecting portion (61) in the first direction, the connecting portion (61) is located in the catch space (303), and the stopping portion (62) is stopped from moving by the limiting plate (331).

5. The sterile adapter (20) according to claim 4, wherein the elastic member (50) is sleeved over the fourth sleeve (43) and the third sleeve (33).

6. The sterile adapter (20) according to claim 4 or 5, wherein the catch member (60) is threaded to the fourth sleeve (43).

7. The sterile adapter (20) according to any one of claims 4 to 6, wherein the fourth sleeve (43) and the second sleeve (42) protrude from the second bottom (41) on a same side of the second bottom (41).

8. The sterile adapter (20) according to any one of claims 2 to 7, wherein the drive assembly (23) further includes a limiting mechanism (70) formed by a portion of an outer wall surface of the first sleeve (32) and a portion of an inner wall surface of the second sleeve (42) that are mated with each other, neither the outer wall surface of the first sleeve (32) nor the inner wall surface of the second sleeve (42) is cylindrical, and the portion of the limiting mechanism (70) located on the outer wall surface of the first sleeve (32) and mated/contacted with the inner wall surface of the second sleeve (42) has a same cross-section at any position in the first direction, and the portion of the limiting mechanism (70) located on the inner wall surface of the second sleeve (42) and mated/contacted with the outer wall surface of the first sleeve (32) has a same cross-section at any position in the first direction.

9. The sterile adapter (20) according to claim 8, wherein the limiting mechanism (70) includes a limiting groove (71) and a limiting block (72), wherein the limiting groove (71) is disposed on one of the outer wall surface of the first sleeve (31) and the inner wall surface of the second sleeve (42), and the limiting block (72) is disposed on the other one of the outer wall surface of the first sleeve (31) and the inner wall surface of the second sleeve (42), the limiting block (72) being located in the limiting groove (71) and movable relative to the limiting groove (71) in the first direction.

10. The sterile adapter (20) according to claim 9, wherein a plurality of limiting mechanisms (70) are provided, and the plurality of the limiting mechanisms (70) are arranged at intervals around an axis of the first sleeve (32).

11. The sterile adapter (20) according to any one of claims 2 to 10, wherein the drive assembly (23) further includes an orientation mechanism (80) formed by a portion of the outer wall surface of the first sleeve (32) and a portion of the inner wall surface of the second sleeve (42) that are mated with each other, the orientation mechanism (80) has a predetermined angle or position with at least one of a drive input end or a drive output end of the drive assembly (23), and the portion of the orientation mechanism (80) located on the outer wall surface of the first sleeve (32) and mated/contacted with the inner wall surface of the second sleeve (42) has a same cross-section at any position in the first direction, and the portion of the orientation mechanism (80) located on the inner wall surface of the second sleeve (42) and mated/contacted with the outer wall surface of the first sleeve (32) has a same cross-section at any position in the first direction.

12. The sterile adapter (20) according to claim 11, wherein the orientation mechanism (80) includes an orientation groove (81) and an orientation block (82), wherein the orientation groove (81) is disposed on one of the outer wall surface of the first sleeve (32) and the inner wall surface of the second sleeve (42), and the orientation block (82) is disposed on the other one of the outer wall surface of the first sleeve (32) and the inner wall surface of the second sleeve (42), and the orientation block (82) is located in the orientation groove (81) and movable relative to the orientation groove (81) in the first direction.

13. The sterile adapter (20) according to any one of claims 2 to 12, wherein the input member (30) further includes:
a drive disk (34), disposed on a surface of the first bottom (31) facing away from the first sleeve (32), connected to an edge of the first bottom (31), protruding in a direction away from the first sleeve (32), and configured to connect the instrument drive (13) and receive the torque output from the instrument drive (13).

14. The sterile adapter (20) according to any one of claims 2 to 13, wherein at least one of a surface of the second bottom (41) facing the first bottom (31) or a surface of the first bottom (31) facing the second bottom (41) is provided with a positioning groove (411), and the elastic member (50) is limited within the positioning groove (411).

15. The sterile adapter (20) according to any one of claims 1 to 14, further comprising:
a first base body (91) and a second base body (92), wherein the first base body (91) is fixed to the second base body (92) in a stacked manner, a mounting hole is provided on the first base body (91) and the second base body (92) and penetrates the first base body (91) and the second base body (92), the mounting hole has a first flange (911) at the first base body (91) and a second flange (921) at the second base body (92), the drive assembly (23) is mounted in the mounting hole, an outer periphery of the drive assembly (23) is provided with a catch tab, the catch tab is located in a space formed by the first flange (911) and the second flange (921), and a radius of a circumference at which the catch tab is located is greater than a radius of each of the first flange (911) and the second flange (921).

16. The sterile adapter (20) according to claim 15, wherein the outer periphery of the drive assembly (23) is provided with at least two catch tabs, and a connecting line between the at least two catch tabs passes through a drive axis of the drive assembly (23).

17. The sterile adapter (20) according to claim 16, wherein the outer periphery of the drive assembly (23) is provided with two catch tabs, and the two catch tabs are different in at least one of size or arc length.

18. The sterile adapter (20) according to any one of claims 15 to 17, wherein a calibration block is disposed in the mounting hole between the first flange (911) and the second flange (921), the calibration block is adjacent or proximate to the first flange (911), and a distance between the calibration block and the second flange (921) is greater than a thickness of the catch tab.
